**Europäisches Patentamt**

**European Patent Office**

**Office européen des brevets**

(19)

(11) Veröffentlichungsnummer : **0 453 456 B1**

(12) # EUROPÄISCHE PATENTSCHRIFT

(45) Veröffentlichungstag der Patentschrift :
**28.04.93 Patentblatt 93/17**

(51) Int. Cl.⁵ : **C07K 13/00, C12N 15/19, C12N 15/62, A61K 37/66**

(21) Anmeldenummer : **90901567.9**

(22) Anmeldetag : **11.01.90**

(86) Internationale Anmeldenummer :
**PCT/EP90/00063**

(87) Internationale Veröffentlichungsnummer :
**WO 90/08163 26.07.90 Gazette 90/17**

(54) **PDGF-A, PDGF-AA, PDGF-AB, HERSTELLUNGSVERFAHREN UND SIE ENTHALTENDE ARZNEIMITTEL.**

(30) Priorität : **12.01.89 DE 3900770**

(43) Veröffentlichungstag der Anmeldung :
**30.10.91 Patentblatt 91/44**

(45) Bekanntmachung des Hinweises auf die
Patenterteilung :
**28.04.93 Patentblatt 93/17**

(84) Benannte Vertragsstaaten :
**AT BE DE ES FR GB IT NL SE**

(56) Entgegenhaltungen :
**EP-A- 0 177 957**
**EP-A- 0 259 632**
**EP-A- 0 288 307**
**DE-A- 3 725 320**

(73) Patentinhaber : **HOPPE, Jurgen**
**Koellikerstrasse 2**
**W-8700 Wurzburg (DE)**

(72) Erfinder : **HOPPE, Jürgen**
**Koellikerstrasse 2**
**W-8700 Würzburg (DE)**
Erfinder : **WEICH, Herbert, A.**
**Sundgauallee 90**
**W-7800 Freiburg (DE)**

(74) Vertreter : **Boeters, Hans Dietrich, Dr. et al**
**Boeters & Bauer Patentanwälte Bereiteranger 15**
**W-8000 München 90 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Platelet Derived Growth Factor (PDGF) ist ein Hauptmitogen im Serum, das das Wachstum von Fibroblasten und glatten Muskelzellen in vitro fördert. In vivo wird PDGF in den α-Granula der Thrombozyten gespeichert und nach Stimulation der Thrombozyten freigesetzt. Hochgereinigtes PDGF ist ein basisches Protein, das eine beträchtliche Heterogenität hinsichtlich seines Molekulargewichtes aufweist (27.000 bis 31.000 d). Die Gründe für diese Heterogenität sind Alterung, Prozessierung und die Existenz verschiedener Isoformen des Typs AA, AB oder BB. Die biologische Aktivität all dieser Formen wird durch Reduktion der Disulfidbrücken zerstört. Human-PDGF aus Thrombozyten besteht hauptsächlich aus AB-Heterodimeren (Heldin & Westermark 1984; Deuel et al. 1985; Ross et al. 1986).

A minosäuresequenzierungen in Verbindung mit DNA-Sequenzierungen der Gene zeigten, daß A und B homolog sind (Betsholtz et al. 1986). PDGF-B ist nahezu identisch mit dem transformierten Genprodukt P28$^{v-sis}$ des Simian-Sarcoma-Virus (SSV). In SSV-transformierten Zellen wurden entsprechende Homodimere des Typs BB nachgewiesen, die ähnliche Eigenschaften wie thrombozytäres PDGF zeigten. Allerdings wurden diese BB-Dimeren nur zum geringen Teil aus den infizierten Zellen sekretiert. PDGF-AA-Formen werden hingegen effizient von produzierenden Zellen sekretiert (Heldin et al. 1986). Es gibt eine zunehmende Zahl an Hinweisen, daß die drei Isoformen AA, AB und BB unterschiedliche Funktionen wahrnehmen. Für eingehende Untersuchungen war daher die Entwicklung eines Verfahrens nötig, größere Mengen an PDGF-A, PDGF-B, PDGF-AA und PDGF-AB zu erzeugen.

EP-A-0 259 632 beschreibt PDGF-Analoga, die durch Expression in Eukaryotischen Zellen enhalten worden sind.

EP-A-0 288 307 beschreibt PDGF-A-Polypeptide, die durch Expression in Hefe- oder Sängerzellen enhalten worden sind.

Zur Anwendung von PDGF bei der Wundbehandlung vergleiche man EP-A-0 288 307.

## Erfindung

Gemäß einer Ausführungsform betrifft die Erfindung PDGF-A (Monomeres) oder biologisch aktives (wachstumstimulierendes) PDGF-AA (Dimeres), die dadurch herstellbar sind, daß man

I) mit Hilfe von E. coli, umfassend einen Hybridvektor, der aus pEX und Fremd-DNA gebildet ist, die folgendes Fusionsprotein kodiert:

(a) ein Fusionsprotein aus β-Galaktosidase und PDGF-A der folgenden Aminosäuresequenz (i)

```
SIEEAVPAVCKTRTVIYEIPRSQVDPTSANFLIWPPCVEVKRCTGCCNT

SSVKCQPSRVHHRSVKVAKVEYVRKKPKLKEVQVRLEEHLECACATTSL

NPDYREEDTDVR
```
( i )

oder

(b) ein Fusionsprotein gemäß (a), dem eine beliebige Aminosäure fehlt, bei dem eine beliebige Aminosäure durch eine beliebige andere Aminosäure ersetzt ist oder bei dem an einer beliebigen Stelle eine weitere beliebige Aminosäure vorgesehen ist, oder

(c) gemäß (a) oder (b) ein Fusionsprotein, bei dem der C-Terminus oder der N-Terminus um bis zu 14 Aminosäuren deletiert oder ergänzt ist;

ein Fusionsprotein als Dimeres der Monomeren gemäß (a), (b) und/oder (c) exprimiert,

II) das gebildete Fusionsprotein (gegebenenfalls nach Reduktion) chemisch spaltet und ein Monomeres der Aminosäuresequenz gemäß (I) (a) oder ein entsprechendes Monomeres freisetzt, bei dem eine beliebige Aminosäure fehlen kann, eine beliebige Aminosäure durch eine beliebige andere Aminosäure ersetzt sein kann, an einer beliebigen Stelle eine weitere beliebige Aminosäure vorgesehen sein kann und/oder der C-Terminus oder der N-Terminus um bis zu 14 Aminosäuren deletiert oder ergänzt sein kann,

III) die Thiolgruppen durch Sulfonieren schützt,

IV) das geschützte Monomere chromatographisch reinigt,

V) die Sulfogruppen des gereinigten und geschützten Monomeren reduziert und gegebenenfalls ferner

VI) das entschützte Monomere durch Ausbildung von Disulfidbrücken dimerisiert und danach

VII) das gebildete Dimere chromatographisch reinigt.

Gemäß einer weiteren Ausführungsform betrifft die Erfindung biologisch aktives (wachstumstimulierendes) PDGF-AB, das dadurch herstellbar ist, daß man in die vorstehend angeführte Stufe (III) PDGF-A gemäß der vorstehend angeführten Stufe (II) und PDGF-B der folgenden Aminosäuresequenz (ii) oder (iii)

```
              20        30        40        50        60
         IAECKTRTEVFEISRRLIDRTNANFLVWPPCVEVQRCSGCCNNRNVQC    (ii)

       70        80        90        100       110
RPTQVQLRPVQVRKIEIVRKKPIFKKATVTLEDHLACKCETVAAARPVTRSPLN


  1     10        20        30        40        50        60
SLGSLTIAEPAMIAECKTRTEVFEISRRLIDRTNANFLVWPPCVEVQRCSGCCNNRNVQC

       70        80        90        100       110
RPTQVQLRPVQVRKIEIVRKKPIFKKATVTLEDHLACKCETVAAARPVT          (iii)
```

oder einer Aminosäuresequenz gemäß (i) oder (ii), bei der eine beliebige Aminosäure fehlt, bei der eine beliebige Aminosäure durch eine beliebige andere Aminosäure ersetzt ist, bei der an einer beliebigen Stelle eine weitere beliebige Aminosäure vorgesehen ist und/oder bei der der C-Terminus oder der N-Terminus um bis zu 14 Aminosäuren deletiert oder ergänzt ist, einsetzt und gemäß den vorstehend angeführten Stufen (III) bis (VII) verfährt und PDGF-AB gewinnt.

Gemäß einer weiteren Ausführungsform betrifft die Erfindung ein Verfahren zur Herstellung von PDGF-A (Monomeres) und gegebenenfalls biologisch aktivem (wachstumstimulirendem) PDGF-AA (Dimeres), das dadurch *gekennzeichnet* ist, daß man

I) mit Hilfe von E. coli, umfassend einen Hybridvektor, der aus pEX und Fremd-DNA gebildet ist, die folgendes Fusionsprotein kodiert:

(a) ein Fusionsprotein aus β-Galaktosidase und PDGF-A der folgenden Aminosäuresequenz

```
SIEEAVPAVCKTRTVIYEIPRSQVDPTSANFLIWPPCVEVKRCTGCCNT

SSVKCQPSRVHHRSVKVAKVEYVRKKPKLKEVQVRLEEHLECACATTSL

NPDYREEDTDVR
```

oder

(b) ein Fusionsprotein gemäß (a), dem eine beliebige Aminosäure fehlt, bei dem eine beliebige Aminosäure durch eine beliebige andere Aminosäure ersetzt ist oder bei dem an einer beliebigen Stelle eine weitere beliebige Aminosäure vorgesehen ist, oder

(c) ein Fusionsprotein gemäß (a) oder (b), bei dem der C-Terminus oder der N-Terminus um bis zu 14 Aminosäuren deletiert oder ergänzt ist,

ein Fusionsprotein als Dimeres der Monomeren gemäß (a), (b) und/oder (c) exprimiert,

II) das gebildete Fusionsprotein (gegebenenfalls nach Reduktion) chemisch spaltet und ein Monomeres der Aminosäuresequenz gemäß (I) (a) oder ein entsprechendes Monomeres freisetzt, bei dem eine beliebige Aminosäure fehlen kann, eine beliebige Aminosäure durch eine beliebige andere Aminosäure ersetzt sein kann, an einer beliebigen Stelle eine weitere beliebige Aminosäure vorgesehen sein kann und/oder der C-Terminus oder der N-Terminus um bis zu 14 Aminosäuren deletiert oder ergänzt sein kann,

III) die Thiolgruppen durch Sulfonieren schützt,

IV) das geschützte Monomere chromatographisch reinigt,

V) die Sulfogruppen des gereinigten und geschützten Monomeren reduziert und gegebenenfalls ferner

VI) das entschützte Monomere durch Ausbildung von Disulfidbrücken dimerisiert und danach

VII) das gebildete Dimere chromatographisch reinigt.

Gemäß einer weiteren Ausführungsform der Erfindung kann man in Stufe (III) zwei Monomere (PDGF-A) einsetzen, die verschieden sind.

Gemäß einer weiteren Ausführungsform der Erfindung kann man in die Stufe (III) PDGF-A, das bei Stufe (II) anfiel, und PDGF-B einsetzen, wie es vorstehend beschrieben wurde.

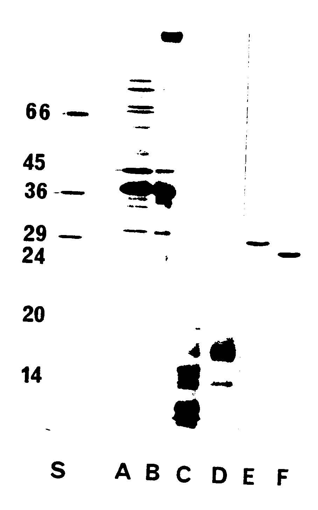

Wie in Abb. 3 gezeigt, fördert der modifizierte pEX-2-Vektor eine Hochexpression des cro-β-gal-PDGF-A-Fusionsproteins. Durch die Deletion von ca. 80 % des lacZ-Gens wird ein kleines Fusionsprotein von 40 000 d gebildet, das mehr als 50 % PDGF-A-Anteil enthält. Trotz dieser günstigen Voraussetzungen sind die Ausbeuten von rPDGF-AA mit 0,2 mg/l Kultur vergleichsweise niedrig. Drei Gründe sind dafür verantwortlich.

1) Die Zellen müssen bei einer geringen Dichte induziert werden und erreichen dadurch nur eine endgültige Dichte von 1,5 $OD_{550}$. Andere Induktions- bzw. Wachstumsbedingungen verschlechterten signifikant die Expression des Fusionsproteins.

2) Die CNBr-Spaltung an der Stelle MSIEE... verläuft nicht vollständig.

3) Durch die Dimerisierung tritt ein Ausbeuteverlust ein.

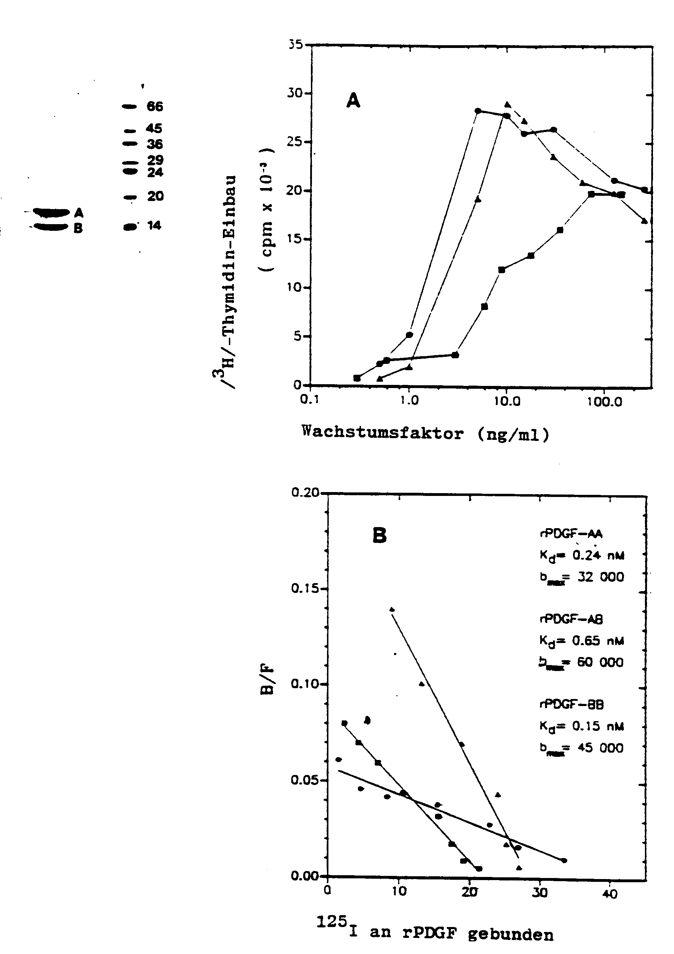

Die biologische Aktivität des rPDGF-AB ist vergleichbar mit der des menschlichen Thrombozyten PDGF und rPDGF-BB aus E. coli. Die Aktivität des rPDGF-AA ist etwa 10-fach geringer (vgl. Abb. 5).

Gemäß einer weiteren Ausführungsform betrifft die Erfindung ein Arzneimittel zur Wundbehandlung, das durch PDGF-A, PDGF-AA und/oder PDGF-AB gemäß der vorstehenden Beschreibung als Wirkstoff gegebenenfalls in Kombination mit üblichen Trägern, Verdünnungsmitteln und/oder ein oder mehreren anderen Wachstumsfaktoren oder die Wundheilung fördernden Substanzen gekennzeichnet ist. Insuline like Growth Faktor (IGF) ist ein Beispiel für einen anderen Wachstumsfaktor.

Nachstehend wird die Erfindung mit Beispielen und Abbildungen naher erläutert. Weitere Einzelheiten zur Herstellung lassen sich Hoppe et al. in Biochem., 28 (1989) 2956-2960 und Eur. J. Biochem., 778 (1989) entnehmen. Im übrigen lassen sich die Sequenzen (i) bis (iii) auch synthetisch herstellen, beispielsweise mit Oligopeptidsynthesemaschinen.

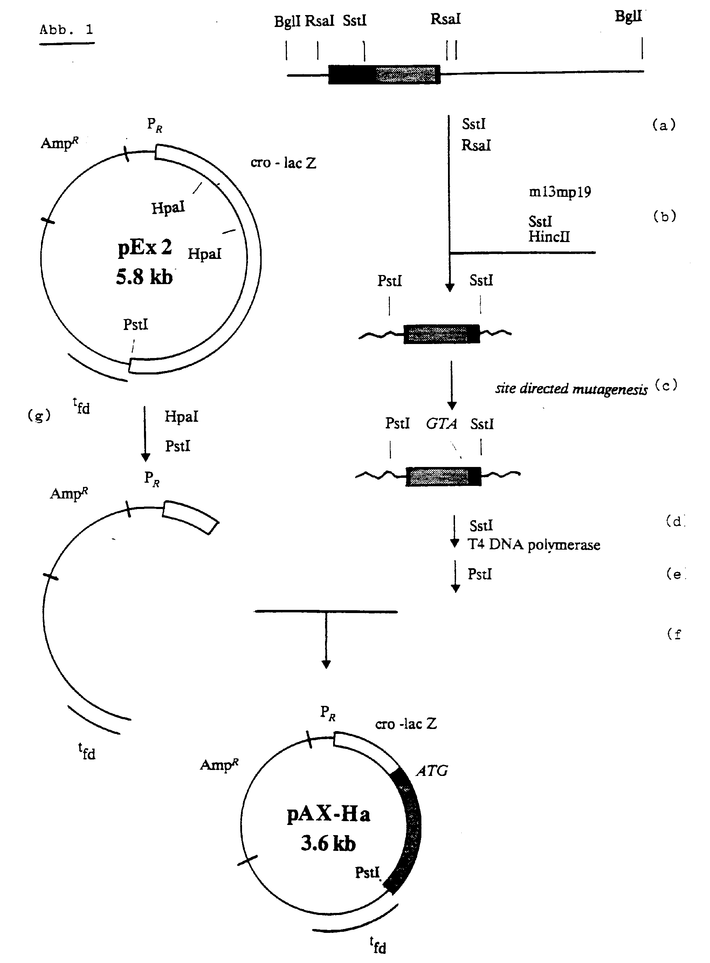

Abb. 1: Strategie für die Konstruktion des Plasmids pAx-HA.

Weiße Kästchen:    cro-lacZ-Gen

Graue Kästchen:    Genbereich für das mature PDGF-A

Schwarze Kästchen:    Genbereiche für Pre-Prosequenzen

Schlangenlinie:    m13mp19-Sequenzen

$P_R$: Bakteriophagen-lambda-promoter; cro-lacZ: Cro-β-Galaktosidase-Fusionsprotein; $t_{fd}$: Phagen-Transkriptions-Terminator.

Abb. 2: Aminosäuresequenz des exprimierten PDGF-A.

A = Alanin, D = Asparaginsäure, E = Glutaminsäure, F = Phenylalanin, G = Glycin, H = Histidin, I = Isoleucin, K = Lysin, L = Leucin, M = Methionin, N = Asparagin, P = Prolin, Q = Glutamin, R = Arginin, S = Serin, T = Threonin, V = Valin, W = Tryptophan, Y = Tyrosin.

Abb. 3: SDS-Gelelektrophorese-Analyse des Expression von PDGF-A in E. coli und der Reinigungsschritte.

A) Lysat aus E.-coli-Zellen

B) Einschlußkörper

C) CNBr-Fragmente

D) Monomeres rPDGF-A nach HPLC-Reinigung

E) Dimeres rPDGF-AA nach Endreinigung

F) Dimeres rPDGF-AB nach Endreinigung

Abb. 4: Bevorzugte Bildung von Heterodimeren aus monomerem rPDGF-A bzw. -B.

Monomeres rPDGF-A und -B wurden in den angegebenen Verhältnissen gemischt und den Renaturierungsbedingungen unterworfen. Nach einem Tag wurden 8 μg durch SDS-Gelelektrophorese analysiert.

Abb. 5: SDS-Gelelektrophorese von gereinigtem rPDGF-AB nach Reduktion mit 2-Merkaptoethanol. Die Banden, die rPDGF-A bzw. rPDGF-B entsprechen, sind angegeben.

Abb. 6: Biologische Aktivitäten von rekombinanten PDGF-Isoformen. (A) Stimulation von [³H]-Thymidin-Einbau in AKR-2B-Maus-Fibroblasten durch rPDGF-AA (schwarze Kästchen), rPDGF-AB (schwarze Kreise) und rPDGF-BB (schwarze Dreiecke). Ein Hintergrund von 2800 cpm wurde abgezogen. (B) Bindung

von $^{125}$I-rPDGF-AA (schwarze Kästchen), $^{125}$I-rPDGF-AB (schwarze Punkte) und $^{125}$I-rPDGF-BB (schwarze Dreiecke) an AKR-2B-Maus-Fibroblasten. rPDGF-BB wurde mit Bolton-Hunter-Reagens markiert. B/F = gebundener/freier Faktor.

Abkürzungen

| HBS : | : Mit Hepes gepufferte Saline |
| PBS : | : Mit Phosphat gepufferte Saline |
| PDGF | : Platelet derived growth factor (= Wachstumsfaktor aus Thrombozyten) |
| rPDGF-AA, -AB, -BB | : rekombinantes PDGF der Isoformen AA, AB und BB |
| p28[v-sis] | : Transformierendes Protein des Simian-Sarcoma -Virus |
| cro-β-gal | : Fusionsprotein aus cro-Repressor und β-Galaktosidase |
| FCS | : Fötales Kälberserum |
| RF | : Replikations-Form |

Materialien

| pMVW | vgl. FEBS Letters, 198 (1986) 344, 345 |
| pEX1 (Plasmid) | Genofit Heidelberg; vgl. auch EMBO J., 3 (1984) 1429-1434 |
| pEX2 (Plasmid) | Genofit, Heidelberg |
| E. coli NF1 | vgl. EMBO J., 3 (1984) 1429-1434 |
| E. coli JC 236 | BioRad |
| E. coli JM 103 | Pharmacia |
| m13mp18 | Pharmacia |
| m13mp19 | Pharmacia |

**Mutagenesekit einschließlich E. coli CJ 236**

**BioRad**

**pPGF-2**　　　　　　　**FEBS Letters, 229 (1987) 243, 244**

Guanidinhydrochlorid und Tris waren von Sigma, Ameisensäure, 2-Propanol, Acetonitril, Trifluoressigsäure von Merck. Sephacryl S-200 war von Pharmacia, die Chromatographiesäule Si-300-polyolbutyl und Ampicillin von Serva, Medium und Supplemente von Gibco und Radiochemikalien von Amersham.

Biologische Systeme

Wachstumstimulierende Aktivität wurde nach Shipley et al. (1984) bestimmt.

Analytische Methoden

Gelelektrophoresen wurden wie beschrieben durchgeführt (13,5-proz. Gele) (Hoppe et al. 1986). Aminosäureanalysen wurden mit einem Analysator (LC2000 von Biotronic) bestimmt. Aminoterminale Sequenzanalysen wurden mit einem Gasphasen-Analysator (470A von Applied Biosystems) bestimmt. Der proteingehalt wurde nach den Methoden von Bradford (1976) oder Redinbaugh (1986) ermittelt.

Beispiel 1: PDGF-A und PDGF-AA

Konstruktion eines Expressionsvektors für PDGF-A-Sequenzen

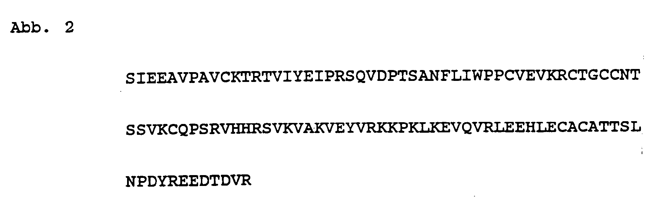

Ein 2 kb langes BglI-Fragment aus Klon pPGF-2 (Hoppe et al. 1987), das den für PDGF-A kodierenden Bereich enthält, wurde mit SstI und RsaI gespalten (Abb. 1 Zeile a). Das so erhaltene 460 b lange Fragment wurde in den Phagen m13mp19 integriert, der zuvor mit SstI und HincII gespalten worden war (Abb. 2 Zeile b). Nach Transformation in den Wirtsstamm JM103 wurden die erhaltenen Kolonien auf korrekte Integration in die Phagen-DNA untersucht. Der Stamm CJ236 (20 ml) wurde dann mit $3 \times 10^6$ der so erhaltenen Phagen infiziert. Danach wurden Uracil enthaltende Phagen wie beschrieben isoliert (BioRad Manual für gezielte Mutagenese). Einzelsträngige Phagen-DNA wurde daraufhin mit dem Primer CGG AGG AAG <u>ATG</u> AGC ATC GAG

GAA G hybridisiert, um das Arginin an Position 76 (AGA) in ein Methionin (ATG) zu mutieren (Abb. 1 Zeile c). Nach Zweitstrangsynthese mittels T4-DNA -Polymerase und Transformation in den Wirtsstamm JM103 wurde der für PDGF-A kodierende Bereich in vier Phagen durch DNA-Sequenzierung analysiert.

Zwei Phagen enthielten den gewünschten Austausch. Einer dieser Phagen wurde verwandt, um RF-DNA zu präparieren. Diese Plasmid-DNA wurde mit SstI verdaut. Überstehende Enden wurden durch T4-DNA -Polymerase-Behandlung entfernt (Abb. 1 Zeile d), wobei ein 460 b langes Fragment durch PstI-Spaltung freigesetzt wurde (Abb. 1 Zeile e). Dieses Fragment wurde in die HpaI/PstI-Stellen des Expressionsvektors pEX-2 integriert (Abb. 1 Zeile f). Dieser HpaI/PstI-Verdau des pEX-2-Plasmides (Abb. 1 Zeile g) führte zu einer Deletion von ca. 2600 b des lacZ-Gens und reduzierte die Größe des cro-β-gal-proteins auf 18 000 d. Die nach der Transfektion in dem Wirtsstamm NF1 erhaltenen Stämme wurde im LB-Medium bei 30 °C bis zu einer optischen Dichte von 0,2 OD (550 nm) kultiviert. Die Produktion des cro-β-gal-PDGF-A -Fusionsproteins wurde durch Erhöhung der Temperatur auf 42 °C induziert. Nach 2 Stunden wurden die Zellen geerntet und Proteine durch SDS-Gelelektrophorese analysiert. Es wurde ein Stamm isoliert, der PDGF-A-Sequenzen hoch exprimiert. Das Plasmid wurde pAX-HA benannt.

### Anzucht von Zellen und Präparation von Einschlußkörpern

E.-coli-Zellen wurden in LB-Medium mit 50 bis 100 μg Ampicillin/ml in 1-1-Kulturen bei 30 °C bis zu einer optischen Dichte von 0,2 OD (440 nm) angezogen und dann bei 42 °C weitere 3 Stunden geschüttelt. Die Zellen wurden durch Zentrifugation (10 min bei 5000 x g) geerntet und in 20 ml Tris-HCl (20 mM) und EDTA (0,5 mM) vom pH 7,8 suspendiert. Für eine typische Präparation wurden 20 bis 30 l Kultur angezogen. Die Zellen wurden durch zwei Passagen durch eine presse (Ribi von Sorvall) bei 20 000 psi oder durch Ultraschallbehandlung aufgeschlossen. Einschlußkörper wurden nach Zugabe von 2 % Triton X100 (Endkonzentration) durch Zentrifugation (10 min bei 6000 x g) erhalten.

### Reduktion und CNBr-Spaltung

Einschlußkörper aus 20 bis 30 l Kultur wurden in 100 ml 50 mM Tris-HCl (50 mM) vom pH 7,8 mit 2 % SDS und 2 % 2-Mercaptoethanol gelöst (ca. 1 h bei 37 °C). Geringe Mengen unlöslichen Materials wurden durch Zentrifugation in 30 min bei 20 000 x g entfernt. Zum Überstand wurden 2 Volumina Aceton bei 0 °C gegeben. Nach 15 min bei 0 °C wurde ein voluminöses Präzipitat abzentrifugiert (10 min bei 6000 x g), das in 80 ml Ameisensäure (100 %) gelöst wurde. Danach wurden 20 ml $H_2O$ hinzugefügt. Unlösliches Material wurde in 1 h bei 50 000 x g entfernt. 1 g CNBr und 200 μl 2-Mercaptoethanol wurden zum Überstand hinzugefügt. Die Reaktion wurde über Nacht bei Raumtemperatur durchgeführt. Die Lösung wurde am Rotationsverdampfer eingetrocknet. Der Rückstand wurde in 80 ml Guanidinhydrochlorid (6 M) aufgenommen, der pH durch Zugabe von 30 % NaOH auf 7,5 eingestellt. Alternativ kann die Spaltung auch in Gegenwart von 6 M Guanidinhydrochlorid durchgeführt werden. Nach Spaltung wird gegen 10 l $H_2O$ dialysiert. Das Dialysat wird mit Guanidinhydrochlorid auf die Endkonzentration von 6 M eingestellt.

### S-Sulfonierung

Zu der erhaltenen Lösung wurden 1 g $Na_2SO_3$ und 0,25 g $Na_2S_2O_6$ hinzugefügt. Die Mischung wurde 5 h lang bei Raumtemperatur belassen. Unlösliches Material wurde durch Zentrifugation entfernt (1 h bei 50 000 x g).

### Reinigung von S-sulfoniertem monomeren rPDGF-A

Die oben erhaltene Lösung wurde auf eine mit Sephacryl S200 gefüllte Säule (Dimension 5 cm ∅ x 100 cm) aufgetragen. Als Elutionsmittel diente Guanidinhydrochlorid (4 M) und Tris-HCl (50 mM) vom pH 7,4. Die Flußrate war 160 ml/h. Es wurden Fraktionen von 15 ml gesammelt. Aliquots der Fraktionen wurden mittels SDS-Gelelektrophorese analysiert. Solche Fraktionen, die Proteine mit einem Molekulargewicht von ca. 17 kd aufwiesen, wurden vereinigt und über Nacht gegen 5 l $H_2O$ dialysiert. Während der Dialyse bildete sich ein Präzipitat, das durch Zugabe von Ameisensäure zu einer Endkonzentration von 10 % weitgehend gelöst werden konnte. Unlösliches Material wurde durch Zentrifugation entfernt (20 min bei 20 0000 x g). Der Überstand (ca. 150 ml) wurde auf eine HPLC-Säule (2 cm ∅ x 25 cm; Umkehrphase: Si-300-polyolbutyl 5 μm) (von Serva) bei einer Flußrate von 2,5 ml/min aufgetragen. Nach Auftrag der Probe wurde mit ca. 2 Säulenvolumina gewaschen. rPDGF-A-Monomer wurde durch einen linearen Gradienten von 10 % Ameisensäure/$H_2O$ gegen 10 % Ameisensäure/60 % 2-Propanol/30 % $H_2O$ während 180 min bei einer Flußrate von 2,5 ml/min eluiert.

rPDGF-A eluierte früh nach 40 bis 50 min. Entsprechende Fraktionen wurden vereinigt und gegen 5 l H$_2$O dialysiert.

Dimerisierung zu rPDGF-AA und Reinigung

S-sulfoniertes monomeres rPDGF-A wurde auf eine Konzentration von 0,4 mg/ml eingestellt. Danach wurde Harnstoff bis zu einer Endkonzentration von 2 M hinzugefügt, danach Glutathion (5 mM) und oxidiertes Glutathion (0,5 mM). Der pH wurde durch Zugabe von Tris-HCl (gegebenenfalls Tris-Base) auf 7,8 eingestellt (Endkonzentration ca. 50 mM). Die Reaktionsmischung wurde 2 Tage lang bei Raumtemperatur belassen.

Dimeres rPDGF-AA wurde durch Ionenaustauscher-Chromatographie gereinigt. Dazu wurde der Dimerisierungsansatz gegen 1 l Na-Azetat (20 mM; pH 5,1), dialysiert und auf eine Säule, die mit Fractogel TSK SP 650 gefüllt war, aufgetragen. Die Säule war in Na-Azetat (20 mM; pH 5,1) äquilibriert, wobei ein Verhältnis von 5 mg Protein/ml Säulenmaterial gewählt wurde. Nach einem Waschvorgang beim pH 5,1 mit Na-Azetat (20 mM) und NaCl (100 mM) wurde beim pH 5,1 mit einem linearen Gradienten von NaCl (100 mM auf 400 mM) in Na-Azetat (20 mM) eluiert. Das Gradientenvolumen betrug das zehnfache Säulenvolumen.

Dimere Formen eluierten später als monomere Spezies.

Beispiel 2: PDGF-AB

rPDGF-B wurde gemäß P 38 34 079.8 = PCT/US 89/..... oder nach folgender Modifikation gewonnen. Das BamH1-DNA-Fragment (2 kb) aus dem Klon pMVW-2 (Weich et al. 1986), das c-sis enthielt, wurde mit umgekehrter Orientierung im Vektor M13mp18 subkloniert. Der 3'-kodierende Bereich der PDGF-B-Kette wurde mit SmaI eliminiert. Zum Eliminieren des 5'-kodierenden Bereichs des c-sis-Gens wurde das Plasmid mit PstI und SalI verdaut, um klebrige 5'- und 3'-Enden für einen Verdau mit Exonuklease II in bekannter Weise zu bilden (Henikoff). Der zweite Strang wurde durch einen S1-Verdau eliminiert; die einzusetzende DNA wurde mit stumpfen Enden versehen, indem man mit DNA-Polymerase-I-Klenow-Fragment auffüllte. Nach der Ligation und Transformation wurden Kolonien, die zu PDGF-B homologe Sequenzen umfaßten, selektiert und sequenziert, um das Ausmaß des Verdaus durch Exonuklease III zu bestimmen. Es wurde ein Plasmid isoliert, das das ATG-Startkodon der SpHI-Stelle der M13mp18-Mehrfachklonierstelle im Leserahmen mit der PDGF-B-Sequenz enthielt. Arg110 in der Sequenz von maturem PDGF wurde mit Hilfe einer gezielten Mutagenese (site directed mutagenesis) in ein Stopkodon gemäß dem BioRad-Protokoll mutiert.

Der Stamm CJ236 (20 ml) wurde mit 3 x 10$^6$ Phagen von M13mp18 infiziert, und U-haltige Phagen wurden in bekannter Weise isoliert. Die Einzelstrangphagen-DNA wurde mit dem Primer CGG CCT GTG ACC <u>TGA</u> AGC CCG G hybridisiert, um Arg110 in ein Stopkodon zu mutieren. Der zweite Strang wurde nach Zugabe von 14-Polymerase synthetisiert. Nach dem Sequenzieren wurden mehrere Stämme erhalten, die ein Stopkodon an der Position 110 enthielten. Von einem dieser Stämme wurde RF-DNA hergestellt und teilweise mit SpHI verdaut. Vorstehende 3'-Enden der SpHI-Stelle wurden in stumpfe Enden durch eine T4-Polymerase-Behandlung überführt. Ein Fragment mit 350 Basenpaaren, das die PDGF-B-Sequenz enthielt, wurde durch EcoRI-Verdau erhalten; dieses Fragment wurde in die EcoRI/EcoRV-Stelle von pEX-2 ligiert. Die Herstellung von monomerem rPDGF-B erfolgte analog zur Herstellung von rPDGF-A.

Für die Herstellung von rPDGF-AB wurden die monomeren Formen rPDGF-A und rPDGF-B in aquimolaren Mengen gemischt (Endkonzentration 0,4 mg/ml Protein). Für die Dimerisierungsbedingungen und die Aufreinigung vergleiche man Beispiel 1.

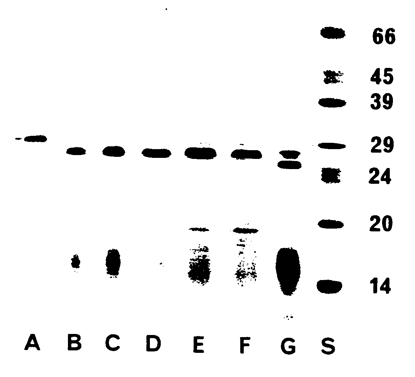

Die Renaturierungsausbeute ist deutlich höher als bei rPDGF-AA (vgl. <u>Abb. 4)</u>.

Die biologische Aktivität des rPDGF-AB ist vergleichbar mit der des menschlichen Thrombozyten-PDGF und rPDGF-BB aus E. coli (vgl. <u>Abb. 6)</u>.

Tabelle 1: Aminosäureanalyse von rPDGF-Isoformen

Die Werte in **Klammern** bezeichnen die Anzahl von Aminosäureresten, die aus bekannten Sequenzen hergeleitet wurden. Die Hydrolyse wurde 24 h lang durchgeführt; Cystein wurde als Cysteinsäure bestimmt. Es wurde keine Korrektur für Abbau oder unvollständige Hydrolyse vorgenommen. Die Werte sind Mittelwerte aus vier Bestimmungen.

| Amino-säure | Menge in | | |
| --- | --- | --- | --- |
| | rPDGF-AA | rPDGF-BB | rPDGF-AB |
| | mol/mol | | |
| Asp | 14 (14) | 16 (16) | 15.0 (15) |
| Thr | 14.3 (16) | 14.7 (16) | 14.9 (16) |
| Ser | 14.3 (16) | 6.1 (6) | 10.9 (11) |
| Glu | 27.3 (28) | 24.6 (24) | 26 (26) |
| Pro | 16.7 (16) | 12.3 (14) | 15.4 (14) |
| Gly | 3.1 (2) | 3.5 (2) | 4.1 (2) |
| Ala | 12.1 (12) | 14.6 (14) | 13.1 (13) |
| Cys | 15.5 (16) | 16.3 (16) | 13.1 (16) |
| Val | 27.3 (30) | 19.6 (24) | 25.0 (27) |
| Ile | 8.2 (8) | 10.4 (12) | 9.3 (10) |
| Leu | 10.6 (10) | 11.9 (12) | 10.9 (11) |
| Tyr | 4.4 (6) | 0.3 (0) | 2.1 (3) |
| Phe | 1.9 (2) | 5.4 (6) | 3.3 (4) |
| Lys | 17.2 (18) | 12.7 (14) | 15.0 (16) |
| His | 5.4 (6) | 1.9 (2) | 3.7 (4) |
| Arg | 16.8 (18) | 20.7 (24) | 19.1 (21) |

Tabelle 2: NH₂-Terminus-Sequenzanalysen von rPDGF-AB

Es wurden 20 µg rPDGF-AB für eine Gas/Flüssig-Sequenzierung verwendet.

| Zyklus | ermittelte Aminosäure | | Sequenz am NH₂-Terminus | |
|--------|------------------------|---|-------------------------|---|
| | | | A-Kette | B-Kette |
| | (pmol) | | | |
| 1 | S | I | S | I |
| 2 | I | A | I | A |
| 3 | E | E | E | E |
| 4 | E | — | E | C |
| 5 | A | K | A | K |
| 6 | V (165) | T (195) | V | T |
| 7 | P (193) | R (98) | P | R |
| 8 | A (220) | T (210) | A | T |
| 9 | V (88) | E (150) | V | E |
| 10 | — | V | C | V |

Literatur

Betsholtz, C., Johnsson, A., Heldin, C.-H., Westermark, B., Lind, P., Urdea, M.S., Eddy, R., Shows, T.B., Philpott, K., Mellor, P.L., Knott, T.J. & Scott, J. (1986) Nature, 320, 695-699.

Bradford, M.B. (1976) Anal. Biochem. 72, 248-253.

Deuel, T.F., Tong, B.D. & Huang, J.S. (1985) Current Topics in Cellular Regulation, 26, 51-61.

Heldin, C.-H. & Westermark, B. (1984) Cell, 37, 9-20.

Heldin, C.-H., Johnsson, A., Wennergren, S., Wernstedt, C., Betsholtz, C. & Westermark, B. (1986) Mature, 319, 511-514.

Henikoff, S. (1984) Gene 28, 351-359.

Hoppe, J., Gatti, D., Weber, H. & Sebald, w. (1986) Eur. J. Biochem., 155, 259-264.

Hoppe, J., weich, H.A. & Eichner, W. Patentanmeldung

Hoppe, J., Schumacher, L., Eichner, W. & Weich, H.A. (1987) FEBS Lett., 223, 243-246.

Redinbaugh, M.G. & Turley, R.B. (1986) Analyt. Biochem. 153, 267-271.

Ross, R., Raines, E.W. & Bowen-Pope, D.F. (1986) Cell, 46, 155-169.

Shipley, G.D., Childs, C.B., Volkenant, M.E. & Moses, H.L. (1984) Cancer Res., 44, 710-716.

Stanley, K.K. & Luzio, J.P. (1984) EMBO J., 3, 1429-1434.

Weich, W., Sebald, W., Schairer, H.-U. & Hoppe, J. (1986) FEBS Lett., 198, 344-348.

Patentansprüche

1.  PDGF-A (Monomeres) oder biologisch aktives (wachstumstimulierendes) PDGF-AA (Dimeres), dadurch herstellbar, daß man

    I) mit Hilfe von E. coli, umfassend einen Hybridvektor, der aus pEX und Fremd-DNA gebildet ist, die folgendes Fusionsprotein kodiert:

    (a) ein Fusionsprotein aus β-Galaktosidase und PDGF-A der folgenden Aminosäuresequenz (i)

SIEEAVPAVCKTRTVIYEIPRSQVDPTSANFLIWPPCVEVKRCTGCCNT

SSVKCQPSRVHHRSVKVAKVEYVRKKPKLKEVQVRLEEHLECACATTSL

NPDYREEDTDVR

(i)

oder
(b) ein Fusionsprotein gemäß (a), dem eine beliebige Aminosäure fehlt, bei dem eine beliebige Aminosäure durch eine beliebige andere Aminosäure ersetzt ist oder bei dem an einer beliebigen Stelle eine weitere beliebige Aminosäure vorgesehen ist, oder
(c) ein Fusionsprotein gemäß (a) oder (b), bei dem der C-Terminus oder der N-Terminus um bis zu 14 Aminosäuren deletiert oder ergänzt ist;
ein Fusionsprotein gemäß (a), (b) und/oder (c) exprimiert,
II) das gebildete Fusionsprotein (gegebenenfalls nach Reduktion)-chemisch spaltet und ein Monomeres der Aminosäuresequenz gemäß (I) (a) oder ein entsprechendes Monomeres freisetzt, bei dem eine beliebige Aminosäure fehlen kann, eine beliebige Aminosäure durch eine beliebige andere Aminosäure ersetzt sein kann, an einer beliebigen Stelle eine weitere beliebige Aminosäure vorgesehen sein kann, und/oder der C-Terminus oder der N-Terminus um bis zu 14 Aminosäuren deletiert oder ergänzt sein kann,
III) die Thiolgruppen durch Sulfonieren schützt,
IV) das geschützte Monomere chromatographisch reinigt,
V) die Sulfogruppen des gereinigten und geschützten Monomeren reduziert und gegebenenfalls ferner
VI) das entschützte Monomere durch Ausbildung von Disulfidbrücken dimerisiert und danach
VII) das gebildete Dimere chromatographisch reinigt.

2.  Biologisch aktives (wachstumstimulierendes) PDGF-AB, dadurch herstellbar, daß man in die Stufe (III) gemäß Anspruch 1 PDGF-A gemäß Anspruch 1 Stufe (II) und PDGF-B der folgenden Aminosäuresequenz (ii) oder (iii)

IAECKTRTEVFEISRRLIDRTNANFLVWPPCVEVQRCSGCCNNRNVQC

(ii)

RPTQVQLRPVQVRKIEIVRKKPIFKKATVTLEDHLACKCETVAAARPVTRSPLN

SLGSLTIAEPAMIAECKTRTEVFEISRRLIDRTNANFLVWPPCVEVQRCSGCCNNRNVQC

RPTQVQLRPVQVRKIEIVRKKPIFKKATVTLEDHLACKCETVAAARPVT       (iii)

oder einer Aminosäuresequenz gemäß (ii) oder (iii), bei der eine beliebige Aminosäure fehlt, bei der eine beliebige Aminosäure durch eine beliebige andere Aminosäure ersetzt ist, bei der an einer beliebigen Stelle eine weitere beliebige Aminosäure vorgesehen ist und/oder bei der der C-Terminus oder der N-Terminus um bis zu 14 Aminosäuren deletiert oder ergänzt ist, einsetzt und gemäß Anspruch 1 Stufen (III) bis (VII) verfährt und PDGF-AB gewinnt.

3. Verfahren zur Herstellung von PDGF-A (Monomeres) und gegebenenfalls biologisch aktivem (wachstumstimulierendem) PDGF-AA (Dimeres), dadurch *gekennzeichnet*, daß man

I) mit Hilfe von E. coli, umfassend einen Hybridvektor, der aus pEX und Fremd-DNA gebildet ist, die folgendes Fusionsprotein kodiert:

(a) ein Fusionsprotein aus β-Galaktosidase und PDGF-A der folgenden Aminosäuresequenz (i)

SIEEAVPAVCKTRTVIYEIPRSQVDPTSANFLIWPPCVEVKRCTGCCNT

SSVKCQPSRVHHRSVKVAKVEYVRKKPKLKEVQVRLEEHLECACATTSL

NPDYREEDTDVR

(i)

oder

(b) ein Fusionsprotein gemäß (a), dem eine beliebige Aminosäure fehlt, bei dem eine beliebige Aminosäure durch eine beliebige andere Aminosäure ersetzt ist oder bei dem an einer beliebigen Stelle eine weitere beliebige Aminosäure vorgesehen ist, oder

(c) ein Fusionsprotein gemäß (a) oder (b), bei dem der C-Terminus oder der N-Terminus um bis zu 14 Aminosäuren deletiert oder ergänzt ist;

ein Fusionsprotein gemäß (a), (b) und/oder (c) exprimiert,

II) das gebildete Fusionsprotein (gegebenenfalls nach Reduktion) chemisch spaltet und ein Monomeres der Aminosäuresequenz gemäß (I) (a) oder ein entsprechendes Monomeres freisetzt, bei dem eine beliebige Aminosäure fehlen kann, eine beliebige Aminosäure durch eine beliebige andere Aminosäure ersetzt sein kann, an einer beliebigen Stelle eine weitere beliebige Aminosäure vorgesehen sein kann und/oder der C-Terminus oder der N-Terminus um bis zu 14 Aminosäuren deletiert oder ergänzt sein kann,

III) die Thiolgruppen durch Sulfonieren schützt,

IV) das geschützte Monomere chromatographisch reinigt,

V) die Sulfogruppen des gereinigten und geschützten Monomeren reduziert und gegebenenfalls ferner

VI) das entschützte Monomere durch Ausbildung von Disulfidbrücken dimerisiert und danach

VII) das gebildete Dimere chromatographisch reinigt.

4. Verfahren nach Anspruch 3, dadurch *gekennzeichnet*, daß man für die Stufe (I) E. coli mit einem Hybridvektor verwendet, der unter Verwendung von pEX, beispielsweise pEX1, pEX2 oder pEX3 gebildet ist.

5. Verfahren nach Anspruch 3 oder 4, dadurch *gekennzeichnet,* daß man bei der Stufe (II) mit Bromcyanid spaltet.

6. Verfahren nach einem der vorhergehenden Ansprüche, dadurch *gekennzeichnet,* daß man bei der Stufe (III) in Gegenwart von Sulfit und Dithionat sulfoniert.

7. Verfahren nach einem der vorhergehenden Ansprüche, dadurch *gekennzeichnet,* daß man bei der Stufe (IV) durch Gelpermeations-Chromatographie und/oder Umkehrphasen-Chromatographie reinigt.

8. Verfahren nach Anspruch 7, dadurch *gekennzeichnet*, daß man bei der Stufe (IV) die Gelpermeations-Chromatographie in Gegenwart eines denaturierenden Agens durchführt, beispielsweise Guanidin-Hydrochlorid, beispielsweise einer Molarität im Bereich von 1 bis 6 M.

9. Verfahren nach einem der vorhergehenden Ansprüche, dadurch *gekennzeichnet*, daß man die Stufen (V) und (VI) gemeinsam durchführt, beispielsweise in Gegenwart

(a) von Harnstoff (beispielsweise einer Molarität von bis zu 4 M) und

(b) Thiolreagenzien, wie

-- 2-Mercaptoethanol (beispielsweise einer Konzentration von bis zu 2 %),

-- Glutathion (beispielsweise einer Molarität von bis zu 100 mM) sowie

-- Dithiothreitol und Dithioerythiol (beispielsweise einer Molarität von bis zu 100 mM).

11

10. Verfahren nach einem der vorhergehenden Ansprüche, dadurch *gekennzeichnet*, daß man bei der Stufe (VII) durch Umkehrphasen-Chromatographie und/oder Ionenaustauscher-Chromatographie reinigt.

11. Verfahren nach einem der vorhergehenden Ansprüche, dadurch *gekennzeichnet*, daß man in die Stufe (III) gemäß Anspruch 3 zwei Monomere gemäß Stufe (II) einsetzt, die verschieden sind.

12. Verfahren zur Herstellung von PDGF-AB, dadurch *gekennzeichnet*, daß man in die Stufe (III) gemäß Anspruch 3 PDGF-A gemäß Anspruch 3 Stufe (II) und PDGF-B gemäß Anspruch 2 einsetzt und gemäß Anspruch 3 Stufen (III) bis (VII) verfährt und PDGF-<u>AB</u> gewinnt.

13. Arzneimittel zur Wundbehandlung, *gekennzeichnet* durch PDGF-A, PDGF-AA und/oder PDGF-AB gemäß einem der Ansprüche 1 und 2 als Wirkstoff gegebenenfalls in Kombination mit üblichen Trägern, Verdünnungsmitteln und/oder ein oder mehreren anderen Wachstumsfaktoren oder die Wundheilung fördernden Substanzen.

14. Arzneimittel nach Anspruch 13, *gekennzeichnet* durch IGF-I (Insuline like Growth Faktor), IGF-II, $\alpha$-TGF, $\beta$-TGF und/oder EGF als anderen Wachstumsfaktor.

## Claims

1. PDGF-<u>A</u> (monomer) or biologically active (growth-stimulating) PDGF-<u>AA</u> (dimer), which can be prepared in such a way that

   I) E. coli, comprising a hybrid vector which is formed from pEX and foreign DNA which encodes the following fusion protein:

   (a) a fusion protein composed of $\beta$-galactosidase and PDGF-A of the following amino-acid sequence (i)

   SIEEAVPAVCKTRTVIYEIPRSQVDPTSANFLIWPPCVEVKRCTGCCNT

   SSVKCQPSRVHHRSVKVAKVEYVRKKPKLKEVQVRLEEHLECACATTSL

   NPDYREEDTDVR                                                          (i)

   or

   (b) a fusion protein according to (a), in which any desired amino acid is absent, in which any desired amino acid has been replaced by any other desired amino acid or in which any desired additional amino acid has been provided at any desired point, or

   (c) a fusion protein according to (a) or (b), in which the C terminus or the N terminus has been deleted or supplemented by up to 14 amino acids;

   is used for the expression of a fusion protein according to (a), (b) and/or (c),

   II) the formed fusion protein (if appropriate after reduction) is chemically cleaved, and a monomer of the amino-acid sequence according to (I) (a) or a corresponding monomer in which any desired amino acid can be absent, any desired amino acid can be replaced by any other desired amino acid, any desired additional amino acid can be provided at any desired point, and/or the C terminus or the N terminus can be deleted or supplemented by up to 14 amino acids, is liberated,

   III) the thiol groups are protected by sulphonation,

   IV) the protected monomer is purified by chromatography,

   V) the sulpho groups in the purified and protected mononer are reduced and, <u>where appropriate</u>, furthermore

   VI) the deprotected monomer is dimerised by formation of disulphide bridges and then

   VII) the formed dimer is purified by chromatography.

2. Biologically active (growth-stimulating) PDGF-<u>AB</u>, which can be prepared by employing in stage (III) according to Claim 1 PDGF-A according to Claim 1 stage (II) and PDGF-B of the following amino-acid se-

quence (ii) or (iii)

IAECKTRTEVFEISRRLIDRTNANFLVWPPCVEVQRCSGCCNNRNVQC

(ii)

RPTQVQLRPVQVRKIEIVRKKPIFKKATVTLEDHLACKCETVAAARPVTRSPLN

SLGSLITIAEPAMIAECKTRTEVFEISRRLIDRTNANFLVWPPCVEVQRCSGCCNNRNVQC

RPTQVQLRPVQVRKIEIVRKKPIFKKATVTLEDHLACKCETVAAARPVT

(iii)

or of an amino-acid sequence according to (ii) or (iii) in which any desired amino acid is absent, in which any desired amino acid is replaced by any other desired amino acid, in which any desired additional amino acid is provided at any desired point and/or in which the C terminus or the N terminus is deleted or supplemented by up to 14 amino acids, and the process according to Claim 1 stages (III) to (VII) is carried out, and PDGF-AB is obtained.

3. Process for the preparation of PDGF-<u>A</u> (monomer) and optionally biologically active (growth-stimulating) PDGF-<u>AA</u> (dimer), characterised in that
I) E. coli, comprising a hybrid vector which is formed from pEX and foreign DNA which encodes the following fusion protein:
(a) a fusion protein composed of β-galactosidase and PDGF-A of the following amino-acid sequence (i)

SIEEAVPAVCKTRTVIYEIPRSQVDPTSANFLIWPPCVEVKRCTGCCNT

SSVKCQPSRVHHRSVKVAKVEYVRKKPKLKEVQVRLEEHLECACATTSL

NPDYREEDTDVR                                                         (i)

or
(b) a fusion protein according to (a), in which any desired amino acid is absent, in which any desired amino acid has been replaced by any other desired amino acid or in which any desired additional amino acid has been provided at any desired point, or
(c) a fusion protein according to (a) or (b), in which the C terminus or the N terminus has been deleted or supplemented by up to 14 amino acids;
is used for the expression of a fusion protein according to (a), (b) and/or (c),
II) the formed fusion protein (if appropriate after reduction) is chemically cleaved, and a monomer of the amino-acid sequence according to (I) (a) or a corresponding monomer in which any desired amino acid can be absent, any desired amino acid can be replaced by any other desired amino acid, any desired additional amino acid can be provided at any desired point, and/or the C terminus or the N terminus can be deleted or supplemented by up to 14 amino acids, is liberated,

III) the thiol groups are protected by sulphonation,

IV) the protected monomer is purified by chromatography,

V) the sulpho groups in the purified and protected monomer are reduced and, where appropriate, furthermore

VI) the deprotected monomer is dimerised by formation of disulphide bridges and then

VII) the formed dimer is purified by chromatography.

4. Process according to Claim 3, characterised in that used for stage (I) is E. coli with a hybrid vector which is formed by using pEX, for example pEX1, pEX2 or pEX3.

5. Process according to Claim 3 or 4, characterised in that cyanogen bromide is used for cleavage in stage (II).

6. Process according to any of the preceding claims, characterised in that sulphonation in stage (III) is carried out in the presence of sulphite and dithionate.

7. Process according to any of the preceding claims, characterised in that purification in stage (IV) is carried out by gel permeation chromatography and/or reverse phase chromatography.

8. Process according to Claim 7, characterised in that the gel permeation chromatography in stage (IV) is carried out in the presence of a denaturing agent, for example guanidine hydrochloride, for example of a molarity in the range from 1 to 6 M.

9. Process according to any of the preceding claims, characterised in that stages (V) and (VI) are carried out together, for example in the presence
   (a) of urea (for example of a molarity of up to 4 M) and
   (b) thiol reagents such as
   2-mercaptoethanol (for example of a concentration of up to 2 %),
   glutathione (for example of a molarity of up to 100 mM) and
   dithiothreitol and dithioerythritol (for example of a molarity of up to 100 mM).

10. Process according to any of the preceding claims, characterised in that purification in stage (VII) is carried out by reverse phase chromatography and/or ion exchange chromatography.

11. Process according to any of the preceding claims, characterised in that two monomers according to stage (II) which are different are employed in stage (III) according to Claim 3.

12. Process for the preparation of PDGF-AB, characterised in that PDGF-A according to Claim 3 stage (II) and PDGF-B according to Claim 2 are employed in stage (III) according to Claim 3, and the process according to Claim 3 stages (III) to (VII) is carried out, and PDGF-AB is obtained.

13. Pharmaceutical for wound treatment, characterised by PDGF-A, PDGF-AA and/or PDGF-AB according to either of Claims 1 and 2 as active substance, where appropriate in combination with customary excipients, diluents and/or one or more other growth factors or substances promoting wound healing.

14. Pharmaceutical according to Claim 13, characterised by IGF-I (insulin like growth factor), IGF-II, $\alpha$-TGF, $\beta$-TGF and/or EGF as other growth factor.

**Revendications**

1. PDGF-A (monomère) ou PDGF-AA biologiquement actif (stimulant la croissance)(dimère), que l'on peut préparer de la manière suivante:
   I) A l'aide de E. coli, comportant un vecteur hybride formé de pEX et d'un ADN étranger, qui code la protéine de fusion suivante :
       (a) une protéine de fusion constituée de $\beta$-galactosidase et de PDGF-A ayant la séquence d'acides aminés suivante (i) :

SIEEAVPAVCKTRTVIYEIPRSQVDPTSANFLIWPPCVEVKRCTGCCNT.

SSVKCQPSRVHERSVKVAKVEYVRKKPKLKEVQVRLEEHLECACATTSL.

NPDYREEDTDVR

ou bien

(b) une protéine de fusion selon (a) à laquelle manque un acide aminé quelconque, dans laquelle un acide aminé quelconque est remplacé par un autre acide aminé quelconque, ou dans laquelle un autre acide aminé quelconque est prévu en un point quelconque, ou bien

(c) une protéine de fusion selon (a) ou (b) dont l'extrémité C-terminale ou N-terminale peut avoir été diminuée ou augmentée d'un nombre d'acides aminés allant jusqu'à 14 ;

on exprime une protéine de fusion selon (a), (b) et/ou (c),

II) on fait subir une dissociation chimique à la protéine de fusion ainsi formée (éventuellement après réduction), et on libère un monomère de la séquence d'acides aminés selon (I) (a) ou un monomère correspondant, dans lequel peut manquer un acide aminé quelconque, dans lequel un acide aminé quelconque peut être remplacé par un autre acide aminé quelconque, en un point quelconque de laquelle on peut prévoir un autre acide aminé quelconque, et/ou dans laquelle l'extrémité C-terminale ou N-terminale peut avoir été diminuée ou augmentée d'un nombre d'acides aminés allant jusqu'à 14,

III) on protège les groupes thiol par sulfonation,

IV) on procède à une purification chromatographique du monomère protégé,

V) on réduit les groupes sulfo des monomères purifiés et protégés, et éventuellement en outre

VI) on dimérise le monomère déprotégé par formation de ponts disulfure, puis

VII) on purifie par chromatographie le dimère formé.

**2.** PDGF-AB biologiquement actif (stimulant la croissance), que l'on peut produire par le fait que l'on introduit dans l'étape (III) selon la revendication 1 un PDGF-A selon l'étape (II) de la revendication 1 et un PDGF-B ayant la séquence d'acides aminés (ii) ou (iii) suivante :

```
              20          30          40          50          60
       IAECKTRTEVFEISRRLIDRTNANFLVWPPCVEVQRCSGCCNNRNVQC
                                                            ( ii )
          70          80          90          100         110
RPTQVQLRPVQVRKIEIVRKGPIFKKATVTLEDHLACKCETVAAARPVTRSPIN
                             -

   1       -    10          20          30          40          50          60
SLGSLTIAEPAMIAECKTRTEVFEISRRLIDRTNANFLVWPPCVEVQRCSGCCNNRNVQC
          70          80          90          100
RPTQVQLRPVQVRKIEIVRKGPIFKKATVTLEDHLACKCETVAAARPVT     ( iii )
```

ou ayant une séquence d'acides aminés selon (ii) ou (iii) dans laquelle manque un acide aminé quelconque, dans laquelle un acide aminé quelconque est remplacé par un autre acide aminé quelconque, dans laquelle un autre acide aminé quelconque est prévu en un point quelconque, et/ou dans laquelle l'extrémité C-terminale ou N-terminale est diminuée ou complétée par un nombre d'acides aminés allant jusqu'à 14, et on procède selon les étapes (III) à (VII) de la revendication 1, et on récupère le PDGF-AB.

3. Procédé pour préparer un PDGF-A (monomère) ou éventuellement un PDGF-AA biologiquement actif (stimulant la croissance) (dimère) caractérisé en ce que :

I) A l'aide de E. coli, comportant un vecteur hybride formé de pEX et d'un ADN étranger, qui code la protéine de fusion suivante :

(a) une protéine de fusion constituée de β-galactosidase et de PDGF-A ayant la séquence d'acides aminés suivante (i) :

SIEEAVPAVCKTRTVIYEIPRSQVDPTSANFLIWPPCVEVKRCTGCCNT

SSVKCQPSRVHHRSVKVAKVEYVRKKPKLKEVQVRLEEHLECACATTSL.

NPDYREEDTDVR

$$(\text{i})$$

ou bien
(b) une protéine de fusion selon (a) à laquelle manque un acide aminé quelconque, dans laquelle un acide aminé quelconque est remplacé par un autre acide aminé quelconque, ou dans laquelle un autre acide aminé quelconque est prévu en un point quelconque, ou bien
(c) une protéine de fusion selon (a) ou (b) dont l'extrémité C-terminale ou N-terminale peut avoir été diminuée ou augmentée d'un nombre d'acides aminés allant jusqu'à 14 ;
on exprime une protéine de fusion selon (a), (b) et/ou (c),
II) on fait subir une dissociation chimique à la protéine de fusion ainsi formée (éventuellement après réduction), et on libère un monomère de la séquence d'acides aminés selon (I) (a) ou un monomère correspondant, dans lequel peut manquer un acide aminé quelconque, dans lequel un acide aminé quelconque peut être remplacé par un autre acide aminé quelconque, en un point quelconque de laquelle on peut prévoir un autre acide aminé quelconque, et/ou dans laquelle l'extrémité C-terminale ou N-terminale peut avoir été diminuée ou augmentée d'un nombre d'acides aminés allant jusqu'à 14,
III) on protège les groupes thiol par sulfonation,
IV) on procède à une purification chromatographique du monomère protégé,
V) on réduit les groupes sulfo du monomère purifié et protégé, et éventuellement en outre
VI) on dimérise le monomère déprotégé par formation de ponts disulfure, puis
VII) on purifie par chromatographie le dimère formé.

4. Procédé selon la revendication 3, caractérisé en ce qu'on utilise pour l'étape (I) un E. Coli ayant un vecteur hybride formé par utilisation de pEX, par exemple pEX-1, pEX-2 ou pEX-3.

5. Procédé selon la revendication 3 ou 4, caractérisé en ce qu'on procède au clivage de l'étape (II) à l'aide de cyanure de brome.

6. Procédé selon l'une des revendications précédentes, caractérisé en ce qu'on procède à la sulfonation de l'étape (III) en présence d'un sulfite et d'un dithionate.

7. Procédé selon l'une des revendications précédentes, caractérisé en ce qu'on procède à la purification dans l'étape (IV) par chromatographie par perméation de gel et/ou par chromatographie en phases inversées.

8. Procédé selon la revendication 7, caractérisé en ce qu'on procède dans l'étape (IV) à la chromatographie par perméation de gel en présence d'un agent de dénaturation, par exemple le chlorhydrate de guanidine, par exemple selon une molarité de 1 à 6 M.

9. Procédé selon l'une des revendications précédentes, caractérisé en ce qu'on met en oeuvre simultanément les étapes 5 et 6, par exemple en présence
(a) d'urée (ayant par exemple une molarité allant jusqu'à 4 M) et
(b) de réactifs de type thiol, tels que:

- le 2-mercaptoéthanol (par exemple à une concentration allant jusqu'à 2 %),
- le glutathion (par exemple à une molarité allant jusqu'à 100 mM) et
- le dithiothréitol et le dithioérythritol (par exemple à une molarité allant jusqu'à 100 mM).

10. Procédé selon l'une des revendications précédentes, caractérisé en ce que dans l'étape (VII), on procède à la purification par chromatographie en phases inversées et/ou par chromatographie par échange d'ions.

11. Procédé selon l'une des revendications précédentes, caractérisé en ce qu'on utilise dans l'étape (III) selon la revendication 3, deux monomères selon l'étape (II), qui sont différents.

12. Procédé pour préparer du PDGF-AB, caractérisé en ce qu'on utilise dans l'étape (III) selon la revendication 3, un PDGF-A selon la revendication 3, étape (II), et un PDGF-B selon la revendication 2, et que l'on procède selon la revendication 3, étapes (III) à (VII), en récupérant le PDGF-AB.

13. Médicament pour le traitement des blessures, caractérisé par du PDGF-A, du PDGF-AA et/ou du PDGF-AB selon l'une des revendications 1 et 2 en tant que principe actif, éventuellement en combinaison avec des excipients ou diluants usuels, et/ou un ou plusieurs autres facteurs de croissance ou substances favorisant la cicatrisation.

14. Médicament selon la revendication 13, caractérisé par de l'IGF-I (Insuline Like Growth Factor, facteur de croissance insulinoïde), de l'IGF-II, de l'$\alpha$-TGF, du $\beta$-TGF et/ou de l'EGF en tant qu'autre facteur de croissance.

Abb. 1

BglI RsaI SstI    RsaI          BglI

SstI             (a)
RsaI

m13mp19

SstI       (b)
HincII

$P_R$

$Amp^R$             cro - lac Z

HpaI

pEx 2
5.8 kb  HpaI

PstI

PstI      SstI

site directed mutagenesis (c)

PstI  GTA  SstI

(g)  $^t$fd

HpaI

PstI

SstI      (d)
T4 DNA polymerase

$P_R$

$Amp^R$

PstI      (e)

(f

$^t$fd

$P_R$    cro -lac Z

$Amp^R$          ATG

pAX-Ha
3.6 kb

PstI

$^t$fd

Abb. 2

SIEEAVPAVCKTRTVIYEIPRSQVDPTSANFLIWPPCVEVKRCTGCCNT

SSVKCQPSRVHHRSVKVAKVEYVRKKPKLKEVQVRLEEHLECACATTSL

NPDYREEDTDVR

66
45
36
29
24
20
14

A
B

35

30  **A**

25

20

15

10

5

0

$/^3H/$-Thymidin-Einbau ( cpm x $10^{-3}$ )

0.1    1.0    10.0    100.0

**Wachstumsfaktor (ng/ml)**

0.20

0.15  **B**

0.10

0.05

0.00

B/F

rPDGF—AA
$K_d$= 0.24 nM
$b_{max}$= 32 000

rPDGF—AB
$K_d$= 0.65 nM
$b_{max}$= 60 000

rPDGF—BB
$K_d$= 0.15 nM
$b_{max}$= 45 000

0    10    20    30    40

$^{125}I$ **an rPDGF gebunden**